(19) Europäisches Patentamt
European Patent Office
Office européen des brevets

(11) **EP 2 287 591 A2**

(12) **EUROPÄISCHE PATENTANMELDUNG**

(43) Veröffentlichungstag:
**23.02.2011 Patentblatt 2011/08**

(51) Int Cl.:
*G01N 21/35* (2006.01)    *G01N 33/00* (2006.01)
*G01N 1/22* (2006.01)

(21) Anmeldenummer: **10173120.6**

(22) Anmeldetag: **17.08.2010**

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**BA ME RS**

(30) Priorität: **17.08.2009   DE 102009037706**

(71) Anmelder: **Opsolution Nanophotonics GmbH**
**34119 Kassel (DE)**

(72) Erfinder: **Sandhagen, Carl**
**34125 Kassel (DE)**

(74) Vertreter: **Beck & Rössig**
**European Patent Attorneys**
**Cuvilliésstrasse 14**
**81679 München (DE)**

(54) **Verfahren und Vorrichtung zur Bestimmung der Konzentration von NO2 in Gasgemischen**

(57) Eine Vorrichtung zur Bestimmung der Konzentration von $NO_2$ in Gasgemischen, insbesondere Abgasen von Dieselmotoren, umfasst eine Lichtquelle zur Ausgabe von Licht in einen Lichtweg; eine Sensoreinrichtung zum Empfang zumindest eines Teils des über den Lichtweg zur Sensoreinrichtung vordringenden Lichtes, wobei der Verlauf des Lichtweges so gewählt ist, dass dieser das zu untersuchende Gas durchsetzt, wobei die Sensoreinrichtung so gestaltet ist, dass diese Signale liefert die das Spektrum des erfassten Lichtes beschreiben; und eine Auswertungsschaltung zur Auswertung der durch die Sensoreinrichtung generierten Signale, wobei die Sensoreinrichtung derart gestaltet ist, die Bestimmung der $NO_2$-Konzentration erfolgt indem die Bestimmung innerhalb eines eingeschränkten spektralen Bereiches erfolgt, in dem eine ausgeprägte absolute oder relative Variation des Absorptionskoeffizienten des $NO_2$ und eine geringe absolute oder relative Variation der weiteren Gase vorliegt. Hierdurch ist es möglich, für die Konzentration von $NO_2$ im Abgas eines Verbrennungsmotors indikative Signale über einen langen Zeitraum hinweg zuverlässig zu generieren.

Figur 4

EP 2 287 591 A2

**Beschreibung**

Gebiet der Erfindung

Die Erfindung betrifft ein Verfahren und eine Vorrichtung zur Bestimmung der $NO_2$-Konzentration in Gasgemischen. Insbesondere richtet sich die Erfindung in diesem Zusammenhang auf ein Verfahren sowie auf eine zu dessen Durchführung vorgesehene Vorrichtung zur Bestimmung der $NO_2$-Konzentration bei Abgasen im Bereich des Abgasweges eines Verbrennungsmotors.

Hintergrund der Erfindung

Aus WO 99/53297 ist eine Vorrichtung bekannt bei welcher die Bestimmung einer $NO_2$-Konzentration auf spektrometrischem Wege unter Einsatz einer LED-Lichtquelle erfolgt.

Bei diesem Ansatz besteht das Problem, dass das Emissionsspektrum und die Intensität der von der LED abgegebenen Strahlung erheblich driftet. Zudem besteht das Problem, dass alle Gase, aber auch Rauch, Russpartikel und anderweitige streuende Partikel, die ebenfalls im blauen Spektralbereich absorbieren oder durch Streuung letztlich bewirken, dass weniger Licht den Lichtsensor erreicht, und es so zu verfälschten Ergebnissen kommt.

Aus der Literaturstelle [Fumikazu Taketani, Megumi Kawai, Kenshi Takahashi, and Yutaka Matsumi, "Trace detection of atmospheric NO2 by laser-induced fluorescence using a GaN diode laser and a diode-pumped YAG laser," Appl. Opt. 46, 907-915 (2007)] ist ebenfalls eine Spektrometereinrichtung bekannt. Diese Spektrometereinrichtung ist so konzipiert, dass diese ein Fluoreszenzsignal nutzt das als solches durch angeregte $NO_2$-Moleküle emittiert wird. Aufgrund des geringen Signals und der geringen Konzentration wird zum Erfassen des Signals ein Photomultiplier benötigt, wobei die Auswertung auf Einzelphotonenereignissen beruht. Eine solche Apparatur ist nur als Laborgerät realisierbar.

Aus US 4001103 ist ein Sensor zur $NO_2$-Detektion auf elektrochemischer Basis mit wässrigem Elektrolyten bekannt. Solche elektrochemische amperometrische Zellen mit 3 Elektroden bzw. elektrochemische Gassensoren können nur im Bereich von 15 bis 90% relativer Feuchte betrieben werden und sind zudem temperaturempfindlich. Eine Anwendung im Abgas eines Verbrennungsmotors ist nur bei entsprechend aufwendiger Aufbereitung des Abgases, welche die Temperatur und den Wassergehalt senkt, möglich.

In [High-sensitivity $NO_2$ sensor based on n-type InP epitaxial layers Wierzbowska et al Optica Applicata Vol. 35, N°3 (2005) pp. 655-662] wird ein resistiver Sensor auf der Basis von InP-Schichten beschrieben. Dieser Sensor besitzt aber eine lange Antwortzeit von 15min bei einer Temperatur von 80°C und benötigt ebenso eine aufwändige Aufbereitung des Abgases.

Aus US 7156966 ist ein Sensor mit Festelektrolyten bekannt, ausgeführt als $ZRO_2$ Multilayerkeramik, welcher NOx nur unspezifisch erfassen kann. Durch die Verwendung eines Festelektrolyten beträgt die Betriebstemperatur mindestens 450°C. Bei Einsatz in einem Fahrzeug stehen die benötigten Messwerte, zudem nur als Summensignal aller NOx, erst einige Minuten nach dem Anlassen des Motors zur Verfügung, obwohl der Sensor zusätzlich elektrisch beheizt wird. Dies hat zur Folge, dass gerade in der emissionsintensiven Kaltstartphase keine Informationen für die Motor- und Katalysatorsteuerung zur Verfügung stehen.

Aus DE000019635977A1 ist die Bestimmung des Beladungszustandes mittels der Nutzung der Änderung der elektrischen Eigenschaften des NOx-Speichermaterials bekannt. Zur Bestimmung der $NO_2$-Konzentration sind solche Methoden nicht geeignet, da diese nur die Bestimmung des aktuellen Beladungszustandes, nicht aber der aktuellen Konzentration ermöglichen. Das Ansprechverhalten des Systems ist zu träge und zu sehr von der Temperatur und der Abgaszusammensetzung abhängig, um aus der Veränderung des Beladungszustandes eine $NO_2$-Konzentration ableiten zu können oder erfindungsgemäß zu verwerten.

Zusammenfassend kann gesagt werden, dass die bekannten $NO_2$-Sensoren nicht die notwendige Sensitivität erreichen oder starken Alterungsprozessen unterliegen bzw. für den Einsatz in motorischen Abgasen nicht geeignet sind. Geeignete Sensoren, wie die in der Abgasanalytik an Motorprüfständen verwendeten FTIR-Spektrometer, sind sehr aufwändig und kostenintensiv.

Aufgabe der Erfindung

Der Erfindung liegt die Aufgabe zugrunde, Lösungen zu schaffen, durch welche es möglich wird hinsichtlich der Konzentration von $NO_2$ im Abgas eines Verbrennungsmotors indikative Signale über einen langen Zeitraum hinweg zuverlässig zu generieren.

Erfindungsgemäße Lösung

Die vorgenannte Aufgabe wird erfindungsgemäß durch eine Vorrichtung mit den in Patentanspruch 1 angegebenen Merkmalen gelöst.

Die erfindungsgemäße Vorrichtung ermöglicht die Konzentrationsbestimmung von $NO_2$ in Gasgemischen wie z.B. Abgasen von Dieselmotoren. Die Bestimmung der $NO_2$ - Konzentration ermöglicht den Betrieb von Motoren in hinsichtlich des Wirkungsgrads und des Kraftstoffverbrauchs optimierter Weise, insbesondere bei Motorsystemen, die im Abgasweg mit einem SCR-Katalysators ausgerüstet sind. Durch die Kenntnis der $NO_2$ - Konzentration vor und hinter dem SCR-Katalysator kann der Motor mit Parametern betrieben werden, welche einen möglichst geringen Kraftstoffverbrauch bewirken aber gleichzeitig eine hohe Konzentration von $NO_2$ im Rohabgas hervorrufen. Andererseits lässt sich aus den Konzentrationswerten der augenblickliche Umsetzungsgrad des SCR-Katalysators bestimmen und somit eine ggf. benötigte Menge an $NH_3$ bzw. die Menge der $NH_3$-Quelle (z.B. eine wässrige Harnstofflösung) vorhersagen. Dies ermöglicht es den SCR-Katalysator im optimalen Betriebspunkt ohne Gefahr eines $NH_3$-Schlupfes zu verwenden und gleichzeitig die Kontrolle und das Einhalten der künftigen gesetzlichen $NO_2$-Grenzwerte im emittierten Abgas und damit die Einhaltung der Grenzwerte in der Luft z.B. gemäß der 22. BImSchV zu gewährleisten.

Die Konzentrationsbestimmung erfolgt erfindungsgemäß nach folgendem Schema:

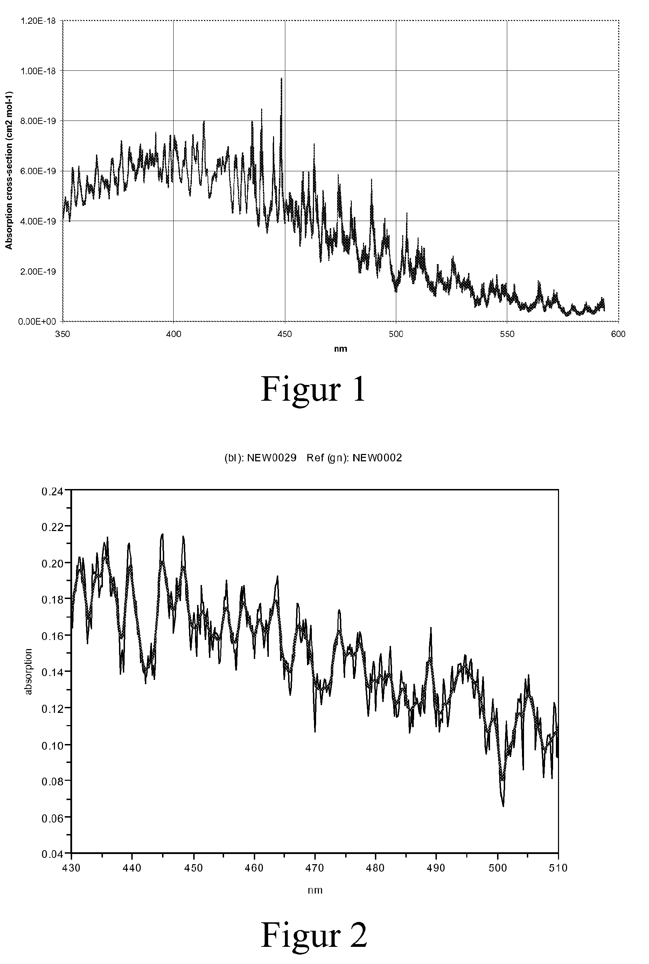

Die spektrale Absorption wird beispielsweise im Abgasstrang eines Verbrennungsmotors ermittelt. Der optische Pfad ist derart angeordnet, dass die Länge des optischen Pfades maximiert bzw. hinsichtlich der Signalqualität oder spektraler Absorption optimiert wird. Beispielsweise kann der optische Pfad so angeordnet sein, dass dieser quer oder längs oder diagonal, aber auch raumdiagonal durch die entsprechenden Kompartimente z. B. einer Uni-Box beispielsweise vor und/oder hinter dem SCR- Katalysator führt. Die selektive Bestimmung der $NO_2$-Konzentration erfolgt innerhalb eines schmalen spektralen Bereiches, in dem die absolute oder relative Variation des Absorptionskoeffizienten des $NO_2$ besonders hoch und die der weiteren Gase vorzugsweise besonders niedrig ist. Die Figur 1 zeigt ein hochaufgelöstes Absorptionsspektrum von $NO_2$ welches die einzelnen Linien der Absorption auflöst.

Weitere Einzelheiten und Merkmale der Erfindung ergeben sich aus der nachfolgenden Beschreibung in Verbindung mit der Zeichnung. Es zeigt:

Figur 1     ein Diagramm zur Veranschaulichung eines hochaufgelösten Absorptionsspektrum von $NO_2$

Figur 2     ein Diagramm zur Veranschaulichung der optischen Absorptionswirkung des Abgases eines Verbrennungsmotors

Figur 3     ein Diagramm zur Veranschaulichung der Differenz der spektralen Absorption des Abgases mit einer Auflösung von 1,5 nm abzüglich der Absorption mit einer Auflösung von 20 nm

Figur 4     die beispielhafte Anordnung des durchstimmbaren Filters, des Sensors sowie eine beispielhafte Ausführung des Strahlenganges und den daraus resultieren optischen Pfad

Figur 5     eine beispielhafte Ausführung einer erfindungsgemäßen Vorrichtung bestehend aus einer mit einer Optik versehenen Lichtquelle, katalytisch ausgestatteten Fenstern, einer Empfängeroptik, ein als durchstimmbares Filter verwendetes Filterrad, einem Strahlungssensor und einer Steuer- und Auswerteeinheit die das Sensorsignal nach einem der beschriebenen Verfahren auswertet

Figur 6     zeigt eine für ein durchstimmbares Filter geeignete Filterscheibe sowie die beispielhafte Ausführung einer Einheit aus Sensor und einer mittels eines Schrittmotors angetriebenen Filterscheibe

Figur 7     zeigt eine für ein durchstimmbares Filter geeignete Filterscheibe mit Referenzmarkierungen zum drehzahlunabhängigen Betrieb sowie die beispielhafte Ausführung einer Einheit aus Sensor und einer mittels eines Getriebemotors angetriebenen Filterscheibe mit zusätzlicher Sensoroptik

Figur 8     zeigt eine für ein durchstimmbares Filter geeignete Filterscheibe mit auf einem eigenen Ring angebrachten Referenzmarkierungen sowie die beispielhafte Ausführung einer Einheit aus Sensor und einer mittels eines Motors angetriebenen Filterscheibe mit zusätzlicher Sensoroptik und zusätzlichen Positionssensor bestehend aus Lichtquelle, Optik und Sensor

Figur 9     beispielhafte Ausführung einer erfindungsgemäßen Vorrichtung mit einer mit einer Optik versehenen Lichtquelle z.B. einer Multichip-LED, katalytisch ausgestatteten und mit einem Luftvorhang geschützten Fenstern, einer Empfängeroptik, ein als durchstimmbares Filter verwendeter Fabry-Pérot-Filter variabler Kavitätslänge, einem Strahlungssensor und einer Steuer- und Auswerteeinheit die das Sensorsignal nach einem der beschriebenen Verfahren auswertet

Figur 10     die Geometrie der Ebene des beweglichen Spiegels bzw. der Membran eines vorteilhaft ausgeführten durchstimmbaren Filter aus DBR- Spiegeln mit tangentialer Anordnung der Verbindungen an eine kreisförmige Filterfläche

Figur 11     die Geometrie eines durchstimmbaren Filters, bei dem zwei Effekte der Änderung der transmittierten Wellenlänge, Änderung des Abstandes der DBR und Änderung des Einstrahlwinkels kombiniert sind

Figur 12     vor der Montage mechanisch justierbare und kalibrierbares Bauteil aus Sendeeinheit und der Empfangseinheit, sowie die Steuer- und Auswerteeinheit zusammengefasst, dabei sind Sender und Empfänger auf einer gemeinsamen optischen Achse fixiert. Die mechanische Verbindung ermöglicht den Gasaustausch mit dem Montageort.

Ausführliche Beschreibung der Figuren

Wie aus der Darstellung nach Figur 1 ersichtlich sind bei Verwendung eines Spektrometer mit beispielsweise 0,3nm Auflösung einzelne Linien nicht mehr erkennbar, es findet eine Mittelung statt, die gemessene absolute oder relative Variation der Absorption sinkt entsprechend. Diese ist aber weiterhin signifikant für $NO_2$. Bei Verwendung eines Spektromers geringer Leistung mit einer Auflösung von beispielsweise 1nm ergibt sich eine stärkere Mittelung, die gemessene absolute oder relative Variation der Absorption sinkt ebenfalls entsprechend. Diese in ihrer Amplitude geminderte Variation ist aber weiterhin signifikant für $NO_2$.

Figur 2 zeigt die optische Absorption des Abgases eines Verbrennungsmotors, die schmale schwarze Linie den Verlauf bei einer Auflösung von 0,3nm, die breite Linie den Verlauf der Absorption bei einer Auflösung bei 1nm. Der Erfindung liegt die Erkenntnis zugrunde, dass für die Identifikation von $NO_2$ im Abgas eines Verbrennungsmotors ein schmaler spektraler Bereich von zum Beispiel 430nm bis 510nm ausreichend ist, auch dann, wenn die Auflösung des Spektrometers groß, z.B. im Bereich von 0,0075 bis 0,1 ,im Verhältnis zum Erfassungsbereich des Spektrometers ist, beispielsweise 1,5nm/40nm. Die Auswahl eines für die Auswertung nach dem erfindungsgemäßen Verfahren geeigneten spektralen Bereiches erfolgt nach folgenden Kriterien, dabei ist der Absorptionsgrad das Verhältnis von in die Messstrecke eingestrahlter Intensität zur am Ende der Messstrecke austretenden Intensität bestimmt in Abhängigkeit bestimmt mit einem Spektrometer der angestrebten Auflösung oder nach Faltung mit einer entsprechenden Funktion welche die gegebene Auflösung in die Auflösung des Spektrometers der Vorrichtung konvertiert. Der Effektivwert des Wechselanteils des Absorptionsgrades des zu detektierenden Stoffes innerhalb des beobachteten bzw. ausgewerteten spektralen Bereiches im Verhältnis zu dessen Mittelwert (3) muss ein z.B. lokales Maximum ergeben, vorzugsweise muss der Effektivwert des Wechselanteils des Absorptionsgrades des zu detektierenden Stoffes im Verhältnis zum Mittelwert des Absorptionsgrades des Stoffgemisches ohne den zu detektierenden Stoff (4) ein z.B. lokales Maximum ergeben. Weiterhin ist der Bereich derart zu wählen, dass die Bedingung (5) dass der Effektivwert des Wechselanteils des Absorptionsgrades des Stoffgemisches ohne den zu detektierenden Stoff innerhalb des beobachteten bzw. ausgewerteten spektralen Bereiches den Wert von 0,61 oder $e^{-\frac{1}{2}}$ mal dem Effektivwert des Wechselanteils des Absorptionsgrades des zu detektierenden Stoffes nicht überschreitet.

$$\overline{a}_x = \frac{1}{\lambda_2 - \lambda_1} \int_{\lambda_1}^{\lambda_2} a_x(\lambda)\, d\lambda \qquad (1)$$

$$\overline{a}_{g-x} = \frac{1}{\lambda_2 - \lambda_1} \int_{\lambda_1}^{\lambda_2} a_{g-x}(\lambda)\, d\lambda \qquad (2)$$

$$\frac{\tilde{a}_{xeff}}{\overline{a}_x} = \frac{\sqrt{\frac{1}{\lambda_2 - \lambda_1} \int_{\lambda_1}^{\lambda_2} \left(a_x(\lambda) - \overline{a}_x\right)^2 d\lambda}}{\frac{1}{\lambda_2 - \lambda_1} \int_{\lambda_1}^{\lambda_2} a_x(\lambda) d\lambda} \qquad (3)$$

$$\frac{\tilde{a}_{xeff}}{\overline{a}_{g-x}} = \frac{\sqrt{\frac{1}{\lambda_2 - \lambda_1} \int_{\lambda_1}^{\lambda_2} \left(a_x(\lambda) - \overline{a}_x\right)^2 d\lambda}}{\frac{1}{\lambda_2 - \lambda_1} \int_{\lambda_1}^{\lambda_2} a_{g-x}(\lambda) d\lambda} \qquad (4)$$

$$\frac{\tilde{a}_{g-xeff}}{\tilde{a}_{xeff}} = \frac{\sqrt{\frac{1}{\lambda_2 - \lambda_1} \int_{\lambda_1}^{\lambda_2} \left(a_{g-x}(\lambda) - \overline{a}_{g-x}\right)^2 d\lambda}}{\sqrt{\frac{1}{\lambda_2 - \lambda_1} \int_{\lambda_1}^{\lambda_2} \left(a_x(\lambda) - \overline{a}_x\right)^2 d\lambda}} \le e^{-\frac{1}{2}} \qquad (5)$$

$a_x$:      Absorptionsgrad des zu detektierenden Stoffes innerhalb der Messstrecke

$a_{g-x}$:      dito Absorptionsgrad des Gemisches ohne den zu detektierenden Stoff

$\overline{a}_x$:      Mittelwert des Absorptionsgrades des zu detektierenden Stoffe

$\overline{a}_{g-x}$:      Mittelwert des Absorptionsgrades des Gemisches ohne den zu detektierenden Stoffe

$\dfrac{\tilde{a}_{xeff}}{\overline{a}_x}$      :      Verhältnis Effektivwert des Wechselanteils des Absorptionsgrades zum Mittelwert

$\dfrac{\tilde{a}_{xeff}}{\overline{a}_{g-x}}$      :      Verhältnis Effektivwert des Wechselanteils des Absorptionsgrades zum Mittelwert

$\dfrac{\tilde{a}_{g-xeff}}{\tilde{a}_{xeff}}$      :      Verhältnis der Effektivwerte der Wechselanteile

Zur Steigerung der Sensitivität oder der Toleranz gegenüber Störsignalen können mehrere entsprechend ausgewählte spektrale Bereiche kombiniert werden. Die Auswahl der Spektralbereiche nach oben beschriebener Methode ermöglicht die Verwendung eines einfachen Spektrometers, welches beispielsweise eine Auflösung von 1nm durch die Verwendung einer Ordnung größer 1 erreicht, dabei durch das Verwenden einer Ordnung größer 1 der erfassbare Bereich beschränkt wird. Ebenso ermöglicht dies die Verwendung eines einfachen durchstimmbaren optischen Filters, welches aufgrund des schmalen benötigten Bereiches nur eine geringe Modulation der Transmissionswellenlänge von beispielsweise nur 3% der mittleren Transmissionswellenlänge erreichen muss.

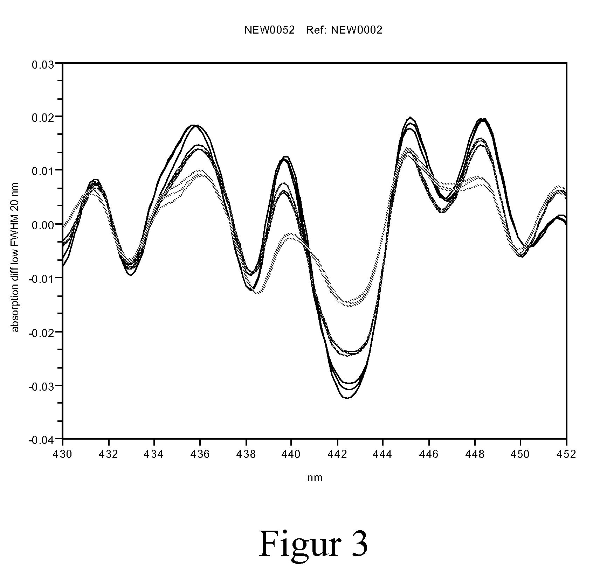

Wird von dem spektralen Signal ein gewichteter Mittelwert oder ein Signal, welches sich bei gröberer Auflösung von beispielsweise 20nm ergibt, subtrahiert, so verbleiben die für die Identifikation von $NO_2$ notwendigen Charakteristika im Spektrum erhalten. Deren Amplitude beträgt z. B. im Bereich um 444 nm noch ¼ der gesamten Absorption um diesen Bereich. Dabei ist dieses Signal aber von allen im Verhältnis zur $NO_2$-Absorption spektral unspezifischen Signalen wie z.B. Offsets oder Driften oder Absorptionen weiterer Bestandteile des Gasgemisches befreit. Die Figur 3 zeigt beispielsweise die Differenz der spektralen Absorption des Abgases eines Verbrennungsmotors im Bereich von 430 bis 452 nm mit einer Auflösung von 1,5 nm abzüglich der Absorption mit einer Auflösung von 20 nm. Dargestellt ist das Signal von 9 verschiedenen Abgasgemischen mit 9 verschiedenen, mittels eines Abgasanalysators bestimmten, $NO_2$-Gehaltes in drei Gruppen um 40, 100 und 160 ppm. Anhand der erfindungsgemäß vorverarbeiteten Signale ist es nun möglich die

Konzentration des $NO_2$-Gehaltes aus den Amplituden bzw. aus den Differenzen benachbarter Minima und Maxima zu bestimmen.

Wie in Verbindung mit Figur 3 veranschaulicht geschieht das erfindungsgemäße Erzeugen der Signale durch Verwendung eines durchstimmbaren Filters und eines Sensors wie z.B. einer Photodiode.

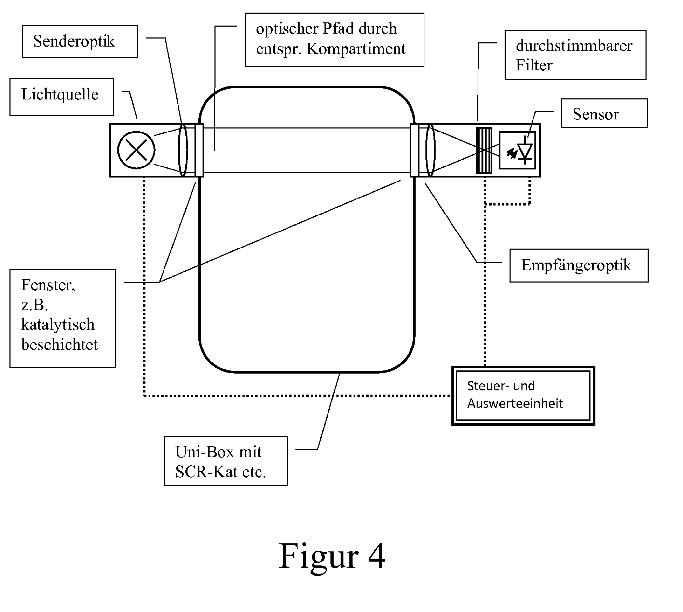

Die Figur 4 zeigt die beispielhafte Anordnung des durchstimmbaren Filters und des Sensors sowie eine beispielhafte Ausführung des Strahlenganges und den daraus resultieren optischen Pfad, dessen Absorption bestimmt wird.

Aus der erfindungsgemäßen Verwendung von nur einem Sensor und eines durchstimmbaren Filters anstelle eines Spektrometers mit Zeilensensor ergeben sich verschiedene Vorteile. Das Signal stammt immer vom gleichen Sensor und ist daher frei von einer PRNU (Photo Response Non-Uniformity), die Auswertung der Signale kann ohne eine vorher bestimmte Lichtempfindlichkeit der Einzelsensoren durchgeführt werden. Das Signal ist ebenfalls frei von DSNU (Dark Signal Non-Uniformity), die Auswertung der Signale kann ohne einen vorher bestimmten Dunkelstrom der Einzelsensoren durchgeführt werden. Ebenfalls entfällt das von CCD- Sensoren bekannte Ausleserauschen oder die Notwendigkeit Driften des Ausleseverstärkers z.B. mittels CDS (Correlated Double Sampling) zu kompensieren, ebenso das durch den Ladungsträgertransport erzeugte Störsignal entfällt. Die gesamte Signalverarbeitungskette ist einfacher und kostengünstiger zu realisieren.

Bei der erfindungsgemäßen Verarbeitung der Sensorsignale ist die Auswertung des Sensorsignals tolerant gegenüber Driften von beispielsweise des Dunkelstroms oder der Empfindlichkeit des Sensors, Driften von beispielsweise des Offsets oder der Arbeitspunkte der Verstärker, gegenüber Driften der Lichtquelle, z.B. hervorgerufen durch Alterung der Quelle, schwankende Betriebstemperatur oder Schwankungen in der Versorgungsspannung und Veränderungen im optischen Pfad z.B. durch ein Verschmutzen der Fenster.

Die Intensität der Strahlung, die den Sensor erreicht ist abhängig von der Wellenlänge, die das durchstimmbare Filter transmittiert sowie von der $NO_2$-Konzentration und dem Extinktionskoeffizienten von $NO_2$, bei der durch den durchstimmbaren Filter ausgewählten Wellenlänge. Wird das Filter periodisch angesteuert, d. h. eine Wellenlänge wiederholend ausgewählt, so ist die Frequenz der Strahlungsintensität und somit das Signal des Sensors das n-fache des Steuersignals und mit diesem verknüpft. Das vom Sensor erzeugte Signal kann nun digitalisiert werden und das zur Durchführung der erfindungsgemäßen Verarbeitung der Sensorsignale notwendige Subtraktionssignal kann beispielsweise mittels einer gewichteten oder ungewichteten Mittelwertbildung oder mittels eines FIR- oder IIR- Filters berechnet werden. Das Differenzsignal ermöglicht die Konzentrationsbestimmung von $NO_2$, in dem die lokalen Minima und Maxima ermittelt werden und deren Differenz gebildet wird. Unterstützend kann dabei auch die strenge Phasenbeziehung vom Steuersignal des durchstimmbaren Filters und des Sensorsignals sowie des weiterverarbeiteten Differenzsignals verwendet werden, beispielsweise durch die digitale Realisierung eines Gated Integrator oder Boxcar Averager. Ebenfalls kann die Auswertung beispielsweise mittels der digitalen Realisierung eines Lock-In-Verstärkers erfolgen oder durch Bestimmung der Amplitude bzw. Bestimmung des RMS oder des Spitze-Spitze-Wertes des Wechselanteils des Sensorsignals.

Vorzugswiese wird das Sensorsignal mit analoger Schaltungstechnik vorverarbeitet., so kann beispielsweise der Wechselspannungsanteil mittels eines Hochpasses oder Bandpasses oder eines Kondensators ausgekoppelt und von der niederfrequenten Komponente getrennt werden, die z. B. Offsets oder Driften oder Absorptionen weiterer Bestandteile des Gasgemisches enthält. Dies entspricht bei der vorher beschriebenen Methode der Subtraktion. Da die Information über die $NO_2$-Konzentration nur bzw. auch im Wechselanteil des Sensorsignals enthalten ist, kann dieses Signal mit einem Wechselspannungsverstärker, auch mit begrenzter Bandbreite, verstärkt werden. Eine Drift, z.B. des Offsets eines solchen Verstärkers, hat keinen Einfluss auf das Wechselsignal, dieser kann daher erheblich kostengünstiger als ein entsprechender Messverstärker für Signale mit Gleichanteil realisiert werden. Durch die strenge Phasenverknüpfung und der Modulation des Sensorsignals durch die Absorptionslinien des $NO_2$ mit einem n- fachen der Frequenz der Ansteuerung des Filters, ist die Frequenz dieser Modulation bekannt bzw. kann vorgegeben werden. Daher ergibt sich die Möglichkeit z. B. ein auf diese Frequenz abgestimmtes Bandpassfilter zur gezielten Unterdrückung anderer, z.B. Störsignale beinhaltender, Frequenzen zu verwenden. Eine einfache und daraus resultierende kostengünstige Möglichkeit der Auswertung des Signals zur Ermittlung der $NO_2$-Konzentration ist beispielsweise die Bestimmung der Amplitude bzw. die Bestimmung des RMS oder des Spitze-Spitze-Wertes des entsprechend vorverarbeiteten Sensorsignals. Das vorverarbeitete Signal kann auch digitalisiert werden und die Auswertung kann beispielsweise durch die digitale Realisierung eines Gated Integrator oder Boxcar Averager erfolgen. Ebenfalls kann die Auswertung beispielsweise mittels der digitalen Realisierung eines Lock-In-Verstärkers durchgeführt werden. Weiterhin besteht die Möglichkeit entsprechende Methoden zur Auswertung auch vollständig analog, z.B. mit geeigneten integrierten Schaltkreisen, zu Realisieren. Vorzugsweise wird die Steuerung der Lichtquelle sowie die Steuerung des durchstimmbaren Filters und die Auswertung der Sensorsignale sowie die Schnittstelle zum Datenbus bzw. der Signalausgang in einer Steuer-und Auswerteeinheit kombiniert. Die Steuerung der Lichtquelle, die Steuerung des durchstimmbaren Filters und die Auswertung der Signale können aber auch in getrennten Einheiten realisiert werden. Diese Einheiten sind dann aber durch eine Signalleitung oder vorzugweise mittels einer Datenbusverbindung, z.B. CAN-Bus miteinander verbunden.

Die Höhe der Signalmodulation durch das Zusammenwirken der Absorptionslinien des $NO_2$ und des durch-

stimmbaren Filters ist abhängig von der $NO_2$-Konzentration und von der Intensität der Lichtquelle bzw. der mittleren, z.B. innerhalb des verwendeten Wellenlängenbereiches gemittelten, Transmission des optischen Pfades. Diese kann z.B. durch eine Belagbildung auf den Fenstern hervorgerufen werden. Zur Kompensation solcher Effekte kann beispielsweise der Wechselanteil des Sensorsignals, das vorverarbeitete Signal als auch das ausgewertete Signal in Relation mit dem Gleichanteil gesetzt werden. Vorzugsweise kann aber auch bei nur wechselsignalgeeigneter Vorverarbeitung des Sensorsignals durch Modulation der Lichtquelle mit einer geeigneten, also dem durch das $NO_2$ modulierten Signal ähnlicher oder n- facher Frequenz ein Wechselsignal erzeugt werden und entsprechend dem Signal bei nicht modulierter Lichtquelle ausgewertet werden. Zu diesem Signal kann dann das vorverarbeitete Signal als auch das ausgewertete Signal in Relation gesetzt werden. Es wird z.B. das Verhältnis von Signalhöhe resultierend aus der Lampenmodulation und der Modulation durch die Linienabsorption des $NO_2$ und des durchstimmbaren Filters bestimmt.

Veränderungen im optischen Pfad, Alterung und Driften der Lichtquelle, Veränderung der Transmissionseigenschaften der Fenster werden somit kompensiert und haben keinen Einfluss auf die Konzentrationsbestimmung.

Mit der Erfindung sind folgende Vorteile verbunden:

Das erfindungsgemäße Verfahren ermöglicht die Verwendung von durchstimmbaren Filtern, eine FWHM von beispielsweise 1,5 nm ist hierbei ausreichend. Daher sind z.B. DBR für den Einsatz in einer entsprechenden Vorrichtung geeignet. Es ist nur ein kleiner spektraler Bereich für selektive Messung nötig, daraus folgt, dass das Stoppband relativ schmal sein kann, z.B. kann die Breite des Stoppbandes 10% der mittleren Wellenlänge des Stoppbandes betragen. Derartige Filter sind einfacher herzustellen als Filter mit breiten Stoppbändern.

Die erfindungsgemäße Bestimmung der $NO_2$-Konzentration ermöglicht durch die nicht medienberührte Messung die sofortige Nutzung und die sofortige Bereitstellung von Messdaten ohne dass ein Aufheizen oder das Erreichen einer Aktivierungstemperatur notwendig ist. Im Gegensatz zu der in DE102007000028A1 beschriebenen Methode ist das System wassertropfentolerant.

Die in WO 99/53297 beschriebene Methode mit blauer LED als Lichtquelle und spektral nicht differenzierenden Sensor bewertet unspezifisch, alle Gase, aber auch Rauch, Russpartikel und streuende Partikel, die auch im blauen Spektralbereich absorbieren oder durch Streuung bewirken, dass weniger Licht den Lichtsensor erreichen werden detektiert bzw. fälschlich als $NO_2$. Auch die Drift des Emissionsspektrum der LED und der Intensität mit der Sperrschichttemperatur wird entsprechend bewertet. Die erfindungsgemäße Bestimmung der $NO_2$-Konzentration ist frei von diesen Nachteilen.

Die erfindungsgemäße Bestimmung der $NO_2$-Konzentration ermöglicht eine schnelle Messung da keine Ionen, wie z.B. in EP0816835 beschrieben, transportiert werden müssen. Daher sind auch kurzzeitige Schwankungen detektierbar. So kann z.B. ein Motorsteuergerät diese Schwankung erfassen und bei der Berechnung der den Motor und die Katalystoren steuernden Parameter berücksichtigen.

Es erfolgt eine direkte Messung der $NO_2$-Konzentration und keine Bestimmung der in einem SCR-Katalysator gespeicherten Ammoniakmenge wie z.B. in DE102007009824A1. Dies ermöglicht die kurzfristige, bedarfsgerechte Einspritzung des Reduktionsmittels (wässrige Harnstofflösung, $NH_3$), dadurch wird eine geringere $NH_3$-Speicherfähigkeit benötigt, der SCR-Katalysator kann kleiner und kostengünstiger dimensioniert werden. Durch die exakte Kontrolle der Umsetzung des SCR-Katalysators und die bedarfsgerechte Dosierung einer $NH_3$-Quelle auch während schneller variabler Lastzustände kann auf einen Ammoniak-Sperrkatalysator verzichtet werden.

Aufgrund der ständigen Messung der integralen Intensität bzw. der gemittelten Intensität über den genutzten Wellenlängenbereich, wird bei schneller Abnahme (z. B. innerhalb von Minuten) der integralen Intensität am Sensor ist ein Defekt am Motor detektierbar. So verweist z.B. viel Ruß auf einen Defekt der Einspritzanlage oder der Luftzuführung, Ölnebel o. ä. zeigt z. B. einen Defekt der Kopfdichtung an, usw. Über geeignete Signale, z.B. über den CAN-Bus kann ein Motorsteuergerät über den Defekt informiert werden. Ebenso kann eine Verschmutzung der Fenster oder ein Belag erkannt und signalisiert werden.

Das schnelle Ansprechverhalten ermöglicht die genaue Kenntnis der $NO_2$-Konzentration auch während transienter Vorgänge wie z.B. Lastwechseln eines Verbrennungsmotors. Diese Messdaten erlauben z.B. mit Hilfe von Modellen chemisch-physikalischer Zusammenhänge die Bestimmung der Konzentration weiterer Komponenten oder weiterer Eigenschaften. Diese Daten können beispielsweise zur Einhaltung von Grenzwerten genutzt oder Parameter zur Verfügung gestellt werden, die die Abgasnachbehandlung optimiert, den Wirkungsgrad eines Verbrennungsmotors mit nachfolgender Abgasnachbehandlung maximiert oder die Betriebskosten einer Anlage zur Energieumwandlung, einer Abgasbehandlungsanlage oder beispielsweise eines Kessels minimiert.

Weitere Ausgestaltung der Erfindung:

Das durchstimmbare Filter kann z.B. als Fabry-Pérot-Filter ausgeführt werden. Dabei geschieht die Auswahl der transmittierten Wellenlänge über den Abstand zwischen den z.B. als Braggspiegel ausgeführten Spiegel. Die transmittierte Wellenlänge ist zusätzlich von dem Einfallswinkel des Lichtes abhängig. Bei der Ausführung als Fabry-Perot-Filter wird der Winkelbereich in dem das durch den optischen Pfad transmittierte Licht auf das Filter fällt vorzugsweise möglichst klein gewählt, d. h. die Brennweite möglichst hoch gewählt. Durch die Auswahl der Größe des Winkelbereiches kann die Bandbreite des Filters gezielt beeinflusst und auf die Anwendung optimiert werden.

Wird das durchstimmbare Filter als Fabry-Pérot-Filter mittels Braggspiegeln ausgeführt, so besitzen diese Spiegel nur einen beschränkten Sperrbereich. Um zu verhindern, dass Strahlung außerhalb des Sperrbereich des Filter diesen erreicht, muss entweder die Quelle derart ausgeführt werden, dass sie kein Licht außerhalb des Sperrbereichs emittiert oder im Strahlengang der Vorrichtung muss ein Bandpass angeordnet werden. Dieser Bandpass kann auf einem eigenen Substrat oder den Fenstern der Komponenten aufgebracht werden. Vorzugsweise wird der Bandpass auf das Substrat des Filters aufgebracht, entweder auf die mit dem Filter beschichtete Seite oder auf die nichtbeschichtete Seite, vorzugsweise auf beide Seiten. Der gesamte Bandpass besteht hierbei aus mehreren einzelnen Bandpässen, Kurzpässen, Langpässen, dabei können alle optischen Komponenten der Vorrichtung als Substrat für die einzelnen Bestandteile des Bandpasses dienen.

Wird die Durchstimmbarkeit des Filters dadurch erreicht, dass die transmittierte Wellenlänge abhängig vom Ort auf dem Filter ist, also über den Ort einen Gradienten aufweist, so ist vorzugsweise der Fokus des durch die Eintrittsöffnung gelangten Lichtes auf die Filterebene gelegt, damit die Strecke entlang des Gradienten der transmittierten Filterwellenlänge möglichst gering ist und somit die Bandbreite des beleuchteten bzw. verwendeten Bereiches des Filters möglichst schmal ist. Ebenso ist hier über die Größe der Strecke eine Anpassung, d.h. eine Vergrößerung der Bandbreite, an die Anwendung möglich.

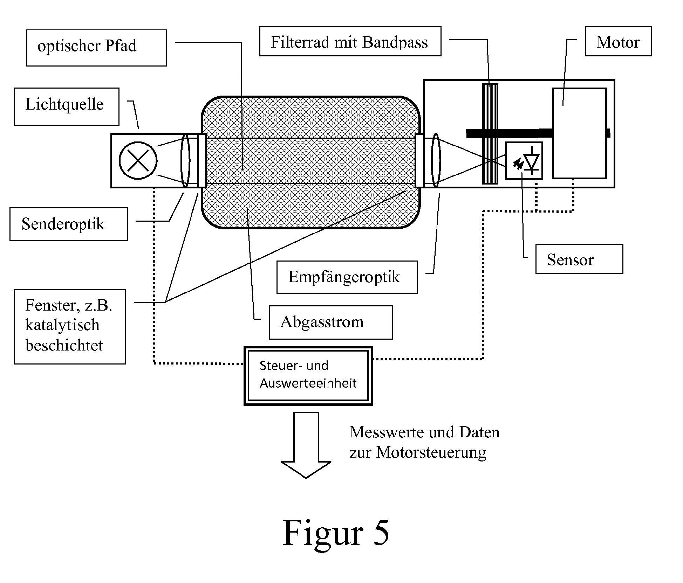

Die Durchstimmung kann auch erreicht werden indem z.B. ein lineares variables Filter (LVF) bzw. ein linearer variabler Bandpass im Strahlengang zumindest zum Teil in Richtung des Gradienten der transmittierten Wellenlänge im Strahlengang bewegt wird. Vorzugsweise ist das Substrat des Filters als Scheibe ausgeführt und drehbar gelagert, wobei die Achse der Lagerung vorzugsweise parallel zur Flächennormalen ausgeführt ist. Als Achse selbst kann z.B. die Achse eines die Scheibe antreibenden Motors, z.B. eines Schrittmotors, eines elektronisch kommutieren Motors oder eines Motors mit Kurzschlussläufer bzw. Ferrarisläufer verwendet werden. Die Figur 5 zeigt eine beispielhafte Ausführung einer erfindungsgemäßen Vorrichtung. Die Durchstimmung kann auch erzielt werden mittels einer linearen Bewegung des Filters z.B. mittels eines Linearmotors oder eines Motors der auf eine Zahn- oder Gewindestange wirkt. Die Bewegung kann auch mittels eines Galvanometers oder eines Schwenkmotors erfolgen.

Wird die Durchstimmbarkeit des Filters dadurch erreicht, dass die transmittierte Wellenlänge abhängig vom Abstand zweier Spiegel oder DBR-Filter ist so ist vorzugsweise der Strahlengang durch den durchstimmbaren Filter so gestaltet, dass der Winkelbereich bzw. der Öffnungswinkel an der Stelle des Filters möglichst gering ist. Dies kann beispielsweise durch eine Optik zwischen Eintrittsöffnung und Filter realisiert werden, deren Abstand von der z.B. als näherungsweise punktförmige Quelle ausgeführten Eintrittsöffnung der Brennweite dieser Optik entspricht. Der Abstand der Spiegel oder der DBR-Filter bzw. die Länge der Kavität des durchstimmbaren Filters kann variiert werden, indem ein oder beide Spiegel oder DBR-Filter beweglich gelagert oder aufgehängt sind. Die Aufhängung wird vorzugsweise mittels Festkörperlagern oder Foliengelenken realisiert, die eine hohe Beweglichkeit in senkrechter Richtung zur Filterebene besitzen und eine möglichst geringe Beweglichkeit, d.h. eine große Steifheit parallel zur Filterebene besitzen. Der Antrieb bzw. die Bewegung, d.h. die Veränderung des Abstandes zwischen Spiegeln bzw. DBR-Filtern kann vorzugsweise elektrisch bzw. elektrostatisch erfolgen, aber auch magnetisch bzw. elektromagnetisch, mittels Gravitation, mittels Beschleunigung oder Trägheitsmomenten erfolgen. Die Variation des Abstandes kann auch mittels des Druckes eines Mediums erfolgen, das auf eine oder auf beide als Membran ausgeführte Spiegel wirkt. Die Bewegung kann aber auch mit einer z.B. thermisch induzierten Geometrieveränderung oder Längenänderung erfolgen..

Die Variation der transmittierten Wellenlänge kann auch dadurch erfolgen, dass ein Filter verwendet wird, dessen transmittierte Wellenlänge abhängig vom Eintrittswinkel der Strahlung ist. Die Durchstimmung des Filters erfolgt dann durch Bewegen des Filterelementes z.B. durch Drehen oder Bewegen auf einer Kreisbahn oder Bewegen eines Spiegels oder Bewegen einer Baugruppe der Vorrichtung in der Art, dass der Eintrittswinkel der Strahlung durch das Filter variabel ist. Vorzugsweise werden hierbei als DBR-Filter ausgeführte Spiegel verwendet.

Die Zuordnung der transmittierten Wellenlänge kann durch eine in den Strahlengang eingekoppelte Linienlichtquelle kontrolliert und korrigiert werden. Die Steuer- und Auswerteeinheit aktualisiert dabei die Tabellen zur Zuordnung eines bestimmen Drehwinkels oder einer bestimmten Spannung oder einer anderen, die vom Filter transmittiere Wellenlänge steuernde Größe, mit der entsprechenden Wellenlänge.

Bei Verwendung eines erfindungsgemäßen Verfahrens zur Auswertung der Signale hat eine Verminderung der Transmission der Fenster keinen Einfluss auf den Messwert. Überschreitet die Absorption der Fenster jedoch einen Wert, bei dem die Intensität der Strahlung am Sensor unter einen vom Sensor und der Signalverstärkung aufgrund des Signal-Rauschabstandes vorgegebenen Wertes fällt, so kann von der Vorrichtung kein Messwert ermittelt werden. Zur Verhinderung dieses Zustandes kann die Vorrichtung mit katalytisch wirkenden Fenstern ausgestattet werden, die vorzugsweise mittels des heißen Abgases aber auch z.B. elektrisch beheizt werden kann. Die Wirkung der katalytischen Fenster, d. h. die Abtragsrate muss nicht zwingend konstant oder stets größer als die Abscheiderate von Verunreinigungen wie beispielsweise Ruß sein. Bei der erfindungsgemäßen Verarbeitung hat eine schwankende Absorption der Fenster keine Auswirkung auf das Messergebnis. Es muss lediglich sichergestellt sein, dass der Grenzwert der Absorption über die Lebensdauer eingehalten wird bzw. nicht innerhalb eines Wartungsintervalls zur manuellen oder automatischen mechanischen oder chemischen Reinigung überschritten wird. Eine weitere Möglichkeit ist die Ausgestaltung der Fenster und des die Fenster umgebenen Raumes derart, dass aufgrund der erzielten Strömungsverhältnisse ein Verschmutzen der Fenster verhindert wird oder vorzugweise ein Abtrag von Abscheidungen von den Fenstern ermöglicht und unterstützt wird. Ein Verschmutzen der Fenster kann auch verhindert werden, indem diese von einem Strom eines partikelfreien oder partikelarmen Gases oder Gasgemisches umspült werden, so dass sich ein Vorhang vor den Fenstern ausbildet und diese von stark partikelhaltigen Gasgemischen, deren $NO_2$-Konzentration bestimmt werden soll, abgeschirmt werden.

Beschreibung eines oder mehrerer Ausführungsbeispiele:

Die Figur 5 zeigt eine beispielhafte Ausführung einer erfindungsgemäßen Vorrichtung. Die Lichtquelle ist mit einer Optik versehen, die ein Teil der abgegebenen Strahlung näherungsweise parallel in den Abgasstrom kollimiert. Der Eintritt in das den Abgasstrom führende Rohr oder Behältnis erfolgt durch katalytisch ausgestattete Fenster, ebenso der Austritt. Die den Abgasstrom passierende Strahlung wird mittels der Empfängeroptik auf das Filterrad fokussiert und der durch die Stellung des Filterrades ausgewählte Bereich vom Sensor detektiert. Die Ansteuerung der Strahlungsquelle, die Ansteuerung des Motors bzw. des durchstimmbaren Filters und die Auswertung der Sensorsignale erfolgt nach einem der beschriebenen Verfahren in der Steuer- und Auswerteeinheit. Diese stellt dann der Motorsteuerung in geeigneter Weise Daten und Messwerte zur Verfügung.

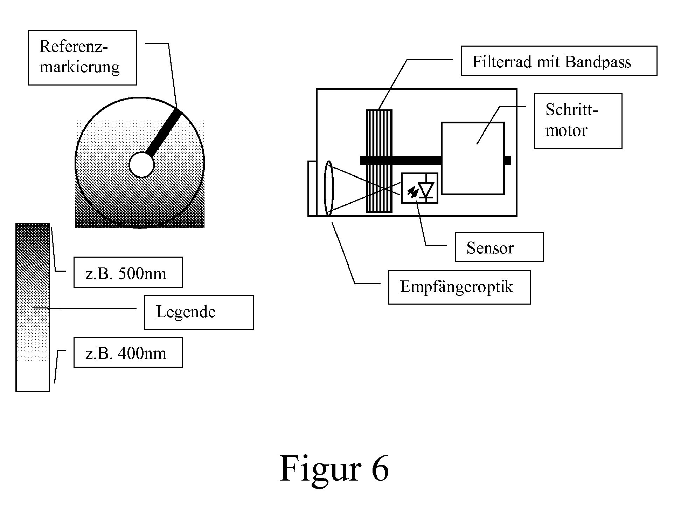

Der durchstimmbare Filter kann entsprechend der Figur 6 mittels einer Filterscheibe mit variabler Transmissionswellenlänge und einem Schrittmotor realisiert werden. Dabei ist auf der Filterscheibe eine Referenzmarkierung angebracht, die keine oder nur sehr wenig Transmission im Bereich des Bandpasses des Filters besitzt. Die Indexmarkierung kann aber auch mittels einer besonders hohen Transmission im Sperrbereich ausgeführt werden. Die Identifikation der Referenzmarkierung erfolgt dann durch das relativ geringe Signal des Sensors bzw. durch das relativ hohe Signal des Sensors, welches sich erkennbar von dem Signal des Sensors unterscheidet, falls sich ein nicht mit der Referenzmarkierung versehener Bereich der Filterscheibe im Strahlengang befindet. In der Steuer- und Auswerteeinheit ist eine bei der Kalibrierung des Systems erstellte Tabelle gespeichert, die die Zuordnung einer bestimmten Anzahl Schritte in eine bestimmte Richtung zu einer transmittierten Wellenlänge ermöglicht. Die Verbindung der Filterscheibe mit der Welle des Motors erfolgt in einem Abstand, der die Montage des Sensors zwischen Filterscheibe und Motor ermöglicht. Der Verlauf der transmittierten Wellenlänge kann entsprechend der Figur 6 quer durch den Mittelpunkt des Filters auf der Filterebene erfolgen.

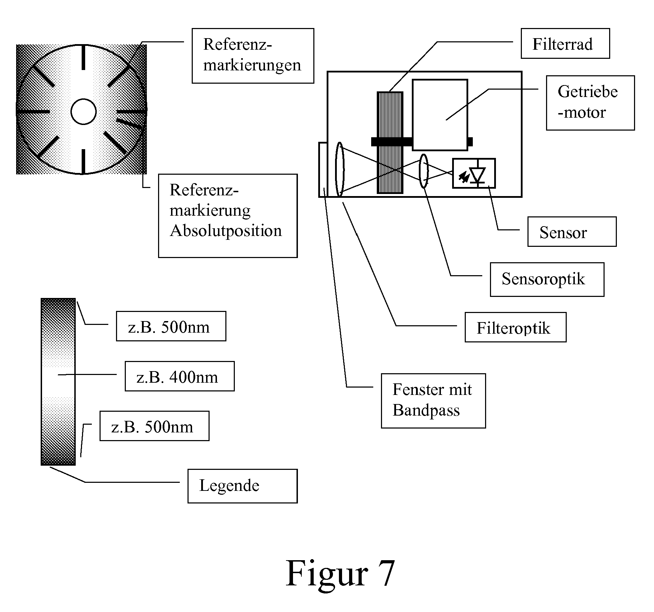

Der durchstimmbare Filter kann auch entsprechend der Figur 7 ausgeführt werden. Die Filterscheibe ist mit mehreren Referenzmarkierungen versehen, dabei unterscheidet sich eine Referenzmarkierung vom Sensor erkennbar von den übrigen Markierungen. Diese dient zur Identifikation der Absolutposition. Mittels dieser Markierungen ist keine konstante Drehzahl nötig und es ist möglich einen freilaufenden d. h. ungeregelten z.B. elektrisch angetriebenen Motor zu verwenden. In der Steuer- und Auswerteeinheit ist eine bei der Kalibrierung des Systems erstellte Tabelle gespeichert, die die Zuordnung einer Position bzw. eines Drehwinkels gemessen zur Referenzmarkierung Absolutposition, sowie die Zuordnung der weiteren Referenzmarkierungen zu dem entsprechenden Winkel ermöglicht. Im Betrieb wird die aktuelle Position mittels der weiteren Referenzmarkierungen bestimmt. Dabei wird von der Steuer- und Auswerteeinheit die Zeit gemessen die zwischen zwei Referenzmarkierungen benötigt wird. Damit ist die Winkelgeschwindigkeit der Filterscheibe innerhalb des von den beiden Referenzmarkierungen eingeschlossenen Winkelbereiches bekannt und der Winkel der Filterscheibe kann mittels des Zeitabstandes zum letzten Durchlaufen einer Referenzmarkierung bestimmt werden. Wird der Antrieb der Filterscheibe entsprechend der Figur 7 mittels eines Getriebemotors realisiert, so ist die Anordnung der Achse am Rand des Getriebemotors möglich. Damit ist die Anordnung des Sensors und einer Sensoroptik neben dem Motor möglich. Die Sensitivität des Sensors kann gesteigert werden, in dem zwischen Filterscheibe und Sensor eine weitere Optik in den Strahlengang eingebracht wird. Diese Optik bündelt das durch die Filterscheibe tretende Licht auf die aktive Fläche des Sensors. Der Verlauf der transmittierten Wellenlänge in Abhängigkeit vom Ort auf der Filterscheibe kann entsprechend der Figur 7 vom Rand des Filters zum Mittelpunkt hin abnehmen und danach wieder zunehmen, wobei das Minimum auch nur in der Nähe des Mittelpunktes liegen kann und das Maxima nur auf einer Seite der Filterscheibe erreicht wird.

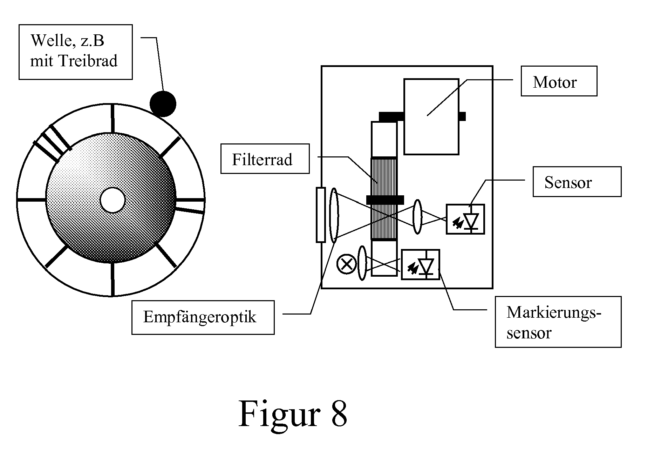

Die Anordnung des Sensors und einer Sensoroptik neben dem Motor ist ebenfalls möglich bei dem Antrieb des Filterrades entfernt von der Mitte, z.B. durch die Übertragung der Drehbewegung am Außenrand der Filterscheibe entsprechend der Figur 8. Dabei kann der Durchmesser der Filterscheibe um einen zusätzlichen Rand, der z.B. die Referenzmarkierungen trägt, vergrößert werden. Dieser Rand muss dabei nicht mit einem Filter versehen sein. Die Detektion der Referenzmarkierungen erfolgt dabei mittels eines weiteren Sensors. Der Verlauf der transmittierten Wellenlänge in Abhängigkeit vom Ort auf der Filterscheibe kann entsprechend der Figur 8, bei der die Bereiche mit der Transmission der langen Wellenlängen näherungsweise im rechten Winkel gegenüberstehen. Allen Beispielen mit motorisch angetriebener Filterscheibe ist gemein, dass die Achse des Motors bzw. die Achse der Filterscheibe nicht parallel zum Strahlengang bzw. zur optischen Achse der Vorrichtung ausgerichtet sein muss.

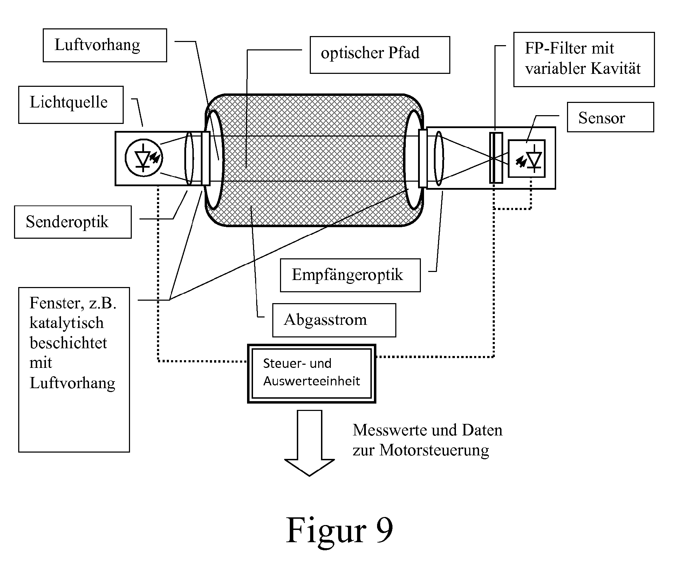

Die Figur 9 zeigt eine weitere beispielhafte Ausführung einer erfindungsgemäßen Vorrichtung. Die Lichtquelle z.B. eine Multichip-LED ist mit einer Optik versehen, die ein Teil der abgegebenen Strahlung näherungsweise parallel in den Abgasstrom richtet. Der Eintritt in das den Abgasstrom führende Rohr oder Behältnis erfolgt ebenso wie der Austritt durch katalytisch ausgestattete Fenster, die zusätzlich von einem Spülgas, welches keine oder deutlich weniger Ablagerungen auf den Fenstern erzeugt wie z.B. Luft, vom Abgas abgeschirmt werden. Die den Abgasstrom passierende Strahlung wird mittels der Empfängeroptik auf den durchstimmbaren Filter fokussiert und der durch den Abstand der Spiegel ausgewählte Bereich vom Sensor detektiert. Wird die Lichtquelle basierend auf LED so ausgeführt, dass diese keine Strahlung außerhalb des Sperrbereiches des durchstimmbaren Filters emittiert, so kann auf einen Bandpass, der die Strahlung auf den Sperrbereich begrenzt verzichtet werden. Die Ansteuerung der Strahlungsquelle, die Ansteuerung des Motors bzw. des durchstimmbaren Filters und die Auswertung der Sensorsignale erfolgt nach einem der beschriebenen Verfahren in der Steuer- und Auswerteeinheit. Diese stellt dann der Motorsteuerung in geeigneter Weise Daten und Messwerte zur Verfügung.

Der durchstimmbare Filter kann dabei auf ein eigenes, für die zu detektierende Strahlung transparentes Substrat aufgebaut werden, aber auch auf dem Sensor, beispielsweise auf dem Fenster des Gehäuses des Sensors oder auf dem Chip aufgebaut werden. Da die zur erfindungsgemäßen Detektion der $NO_2$-Konzentration benötigte Bandbreite nur einige 10nm beträgt, ergeben sich Vorteile zur Ausgestaltung des entsprechenden durchstimmbaren Filters. Wird dieser durchstimmbare Filter mittels DBR-Spiegeln erstellt, muss der Sperrbereich der DBR-Spiegel nur wenig breiter als die benötigte Bandbreite ausgeführt werden. Gleichzeitig ermöglicht dies die Verwendung einer höheren Ordnung des Filters, d.h. der Abstand der Spiegel beträgt ein Mehrfaches der halben Wellenlänge. Da die Halbwertsbreite der durch den Filter transmittierten Strahlung zu der verwendeten Ordnung umgekehrt proportional ist, wird diese durch die Verwendung einer höheren Ordnung verringert. Der durchstimmbare Filter kann daher mit einer, in erster oder zweiter Ordnung breiteren Halbwertsbreite ausgeführt werden als für die Detektion benötigt wird.

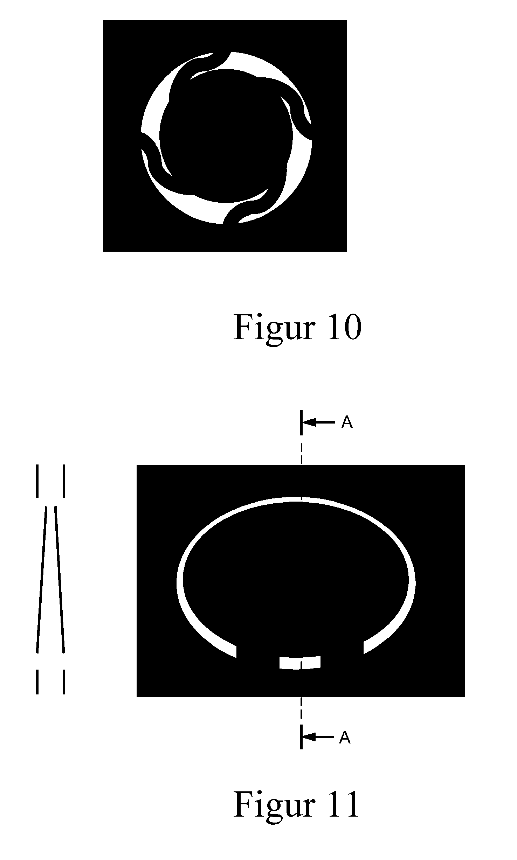

Die Geometrie der Verbindungen ist vorzugsweise derart ausgeführt, dass das Verhältnis von Zwischenräumen und Verbindungen zur Filterfläche möglichst gering und das Verhältnis von Filterfläche zur Gesamtfläche des durchstimmbaren Filters möglichst groß ist. Gleichzeitig wird vorzugsweise die Geometrie der beweglichen Flächen derart, beispielsweise durch eine tangentiale Anordnung der Verbindungen an eine kreisförmige Filterfläche, ausgeführt, dass eine durch die Durchbiegung der Verbindungen in der Aufprojektion scheinbare Verkürzung der Verbindungen durch eine Drehung der beweglichen Filterfläche ausgeglichen wird. Die Figur 10 zeigt ein Ausführungsbeispiel.

Die Figur 11 zeit ein Ausführungsbeispiel eines durchstimmbaren Filters, bei dem zwei Effekte der Änderung der transmittierten Wellenlänge kombiniert sind, einerseits die Änderung durch die Veränderung des Abstandes der DBR andererseits die Änderung durch die Verkippung eines oder beider DBR im Strahlengang. Dazu werden die Verbindungen derart gestaltet, dass eine Drehung der Filterfläche um die zu einer Körperkante oder Tangentialen parallelen Achse möglich ist.

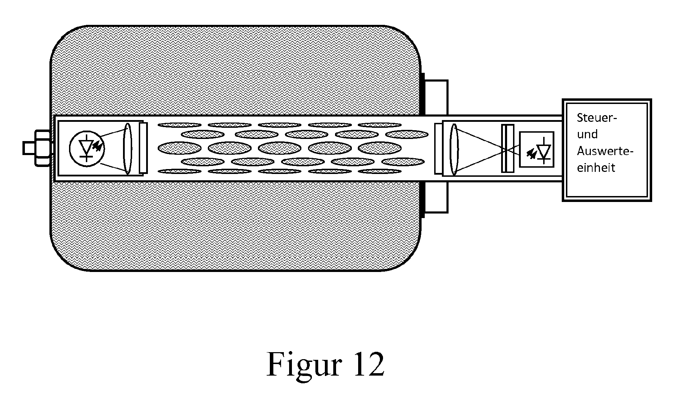

Wird die Sendeeinheit mit der Empfangseinheit zu einem einstückigen Bauteil zusammengefasst, bei dem die Sender und Empfänger z.B. mittels eines mit Löchern versehenen Rohres auf einer gemeinsamen optischen Achse fixiert werden, so kann dieses Bauteil ohne weitere Justage an den Einsatzort montiert werden. Durch den konstanten und vorgegebenen Abstand und der damit festgelegten Küvettenlänge ist die Kalibrierung unabhängig von der Toleranz der Montage des Bauteils beispielsweise in ein Abgasrohr und unabhängig von den Maßtoleranzen der den Abgasstrom führenden Baugruppe. Die Figur 12 zeigt eine beispielhafte Ausführung eines solchen Bauteils. Neben der Sendeeinheit und der Empfangseinheit ist auch die Steuer- und Auswerteeinheit zu einem einstückigen Bauteil zusammengefasst. Die beispielsweise elliptisch ausgeführten Öffnungen ermöglichen den Gasaustausch zwischen dem Raum innerhalb des Bauteils und des umgebenden und abgasführenden Raumes. Die Vorteile eines derartigen kombinierten Bauteils sind die schnelle Installation, der geringe Verkabelungsaufwand, die Robustheit gegen eine unsachgemäße Montage, die Austauschbarkeit und die schnelle Entnahme zu Wartungszwecken. Die Baugruppe kann auch geteilt und mit einer Verriegelung oder Verschraubung verbunden ausgeführt werden, so das zur Montage das Bauteil getrennt wird, dann je ein Teil in gegenüberliegenden Aufnahmen der abgasführenden Baugruppe eingeführt wird und durch Schließen der Verriegelung oder Verschraubung eine formschlüssige Verbindung erzielt wird und die Sende- und Empfangseinheit in der, während der Fertigung zuvor kalibrierten und auf die optische Achse des Bauteils justierten Lage fixiert werden.


**Patentansprüche**

1. Vorrichtung zur Bestimmung der Konzentration von $NO_2$ in Gasgemischen, insbesondere Abgasen von Dieselmotoren, mit:

- einer Lichtquelle zur Ausgabe von Licht in einen Lichtweg;
- einer Sensoreinrichtung zum Empfang zumindest eines Teils des über den Lichtweg zur Sensoreinrichtung vordringenden Lichtes, wobei der Verlauf des Lichtweges so gewählt ist, dass dieser das zu untersuchende Gas durchsetzt, wobei die Sensoreinrichtung so gestaltet ist, dass diese Signale liefert die das Spektrum des erfassten Lichtes beschreiben;
- einer Auswertungsschaltung zur Auswertung der durch die Sensoreinrichtung generierten Signale, wobei die Sensoreinrichtung derart gestaltet ist, die Bestimmung der $NO_2$-Konzentration erfolgt indem die Bestimmung innerhalb eines eingeschränkten spektralen Bereiches erfolgt, in dem eine ausgeprägte absolute oder relative Variation des Absorptionskoeffizienten des $NO_2$ und eine geringe absolute oder relative Variation der weiteren Gase vorliegt.

2. Vorrichtung nach Anspruch 1, **dadurch gekennzeichnet, dass** der vorgenannte schmale spektrale Bereich sich über 430nm bis 510nm erstreckt.

3. Vorrichtung nach Anspruch 1 oder 2, **dadurch gekennzeichnet, dass** die Bestimmung der $NO_2$-Konzentration durch Bestimmung der spektralen Absorption durch Abgas im Abgasstrang eines Verbrennungsmotors innerhalb des vorgenannten eingeschränkten Spektralbereichs erfolgt.

4. Vorrichtung nach wenigstens einem der Ansprüche 1 bis 3, **dadurch gekennzeichnet**, die Sensoreinrichtung ein Spektrometer umfasst dessen Auflösung im Bereich von 1nm liegt, wobei durch die Auflöung einer Ordnung größer 1 der erfassbare Bereich beschränkt wird

5. Vorrichtung nach wenigstens einem der Ansprüche 1 bis 4, **dadurch gekennzeichnet, dass** die Sensoreinrichtung einen durchstimmbaren optischen Filter umfasst, wobei das Filter als Fabry-Pérot-Filter oder durchstimmbarer Fabry-Pérot-Filter mit DBR Spiegeln ausgeführt sein kann und die Geometrie der beweglichen Flächen derart gestaltet sein kann, dass entsprechend Figur 10 eine durch die Durchbiegung der tangentialenVerbindungen des Filters in der Aufprojektion scheinbare Verkürzung der Verbindungen durch eine Drehung der beweglichen Filterfläche ausgeglichen wird, oder das das durchstimmbare Filter entsprechend Figur 5 , Figur 6, Figur 7 oder Figur 8 als Filterscheibe mit variabler Transmissionswellenlänge realisiert ist, wobei der Antrieb z.B. mittels eines Schrittmotors erfolgt und die Identifikation der Wellenlänge bzw. des Drehwinkels mit Hilfe einer oder mehrerer Referenzmarkierungen auf der Filterscheibe ermöglicht wird, wobei die Referenzmarkierungen so gestaltet sein können, dass entsprechend Figur 7 eine oder mehrere Markierungen zur Identifikation der Referenzposition genutzt werden können und die weiteren Markierungen zur Bestimmung der mittleren Winkelgeschwindigkeit innerhalb der Abschnitte zwischen den Markierungen z. B. bei ungeregelten Antrieben, wie z.B. einem elektronisch kommutierten Motor, verwendet werden.

6. Vorrichtung nach Anspruch 5, **dadurch gekennzeichnet, dass** entsprechend Figur 11 zwei Effekte der Änderung der transmittierten Wellenlänge des durchstimmbaren Filters kombiniert sind, einerseits die Änderung durch die Veränderung des Abstandes der DBR, andererseits die Änderung durch die Verkippung eines oder beider DBR im Strahlengang, wobei die Verbindungen derart gestaltet sein kann, dass eine Drehung der Filterfläche um die zu einer Körperkante oder Tangentialen parallelen Achse möglich ist.

7. Vorrichtung nach einem der Ansprüche 5 bis 6, **dadurch gekennzeichnet, dass** der durchstimmbare optische Filter eine Modulation der Transmissionswellenlänge von wenigstens 3% der mittleren Transmissionswellenlänge ermöglicht.

8. Vorrichtung nach wenigstens einem der Ansprüche 1 bis 7, **dadurch gekennzeichnet, dass** die Auswertungsschaltung derart konfiguriert ist, dass durch diese von dem erfassten spektralen Signal ein gewichteter Mittelwert oder ein Signal, welches sich bei gröberer Auflösung von beispielsweise 20nm ergibt, in einem Ausmaß subtrahiert wird, dass im wesentlichen nur die für die Identifikation von $NO_2$ notwendigen Charakteristika im Spektrum verbleiben, wobei die Auswertung auch durch Bestimmung der Amplitude bzw. des RMS oder des Spitze-Spitze-Wert des Wechselanteils des entsprechend vorverarbeiteten Sensorsignals erfolgen kann, oder durch Digitalisierung des vorverarbeiteten Signals, so dass die Auswertung beispielsweise durch digitale Realisierung eines Gated Itegrators oder Boxca Averagers möglich ist, wobei zur Kompensation einer Belagbildung auf den Fenstern oder anderer Veränderungen der Transmissionseigenschaften der Fenster, die im Strahlengang den zu analysierenden Gasstrom vom Sensor abtrennen, der Wechselanteil des Sensorsignals, das vorverarbeitete Signal als auch das ausgewertete Signal in Relation zum Gleichanteil gesetzt werden kann. (vgl. dazu Offenbarung S.10, 11)

9. Vorrichtung nach wenigstens einem der Ansprüche 1 bis 8, **dadurch gekennzeichnet, dass** die Auswertungsschaltung derart konfiguriert ist, dass diese die strenge Phasenverknüpfung und der Modulation des Sensorsignals durch die Absorptionslinien des N02 mit einem n-fachen der Frequenz der Ansteuerung des Filters ähnlich eines Lock-In-Verstärkers nutzt, wobei als Referenzsignal anstelle des Ansteuersignals des durchstimmbaren Filters ein mit diesem phasengekoppeltes Vergleichssignal verwendet werden kann, das sich aus dem wellenlängenabhängigen Absorptionskoeffizienten des $NO_2$ und der durch das durchstimmbare Filter ausgewählten Wellenlänge bei der vorhandenen Halbwertsbreite des durchstimmbaren Filters rechnerisch ergibt und beispielsweise mittels einer vorab berechneten Tabelle innerhalb der Auswerteeinrichtung gespeichert ist.

10. Vorrichtung nach einem der Ansprüche 1 bis 9, **dadurch gekennzeichnet, dass** entsprechend Fig. 12 die Sendeeinheit mit der Empfangseinheit zu einem einstückigen Bauteil zusammengefasst ist, bei dem Sender und Empfänger z.B. mittels eines mit Löchern versehenen Rohres auf einer gemeinsamen optischen Achse fixiert werden, so dass dieses Bauteil ohne weitere Justage an den Einsatzort montiert oder zu Wartungszwecken schnell entnommen werden kann, wobei die Baugruppe auch geteilt sein kann und z.B. zur Verbindung mit einer Verriegelung oder Verschraubung ausgeführt werden kann, so das zur Montage das Bauteil getrennt wird, dann je ein Teil in gegenüberliegenden Aufnahmen der abgasführenden Baugruppe eingeführt wird und durch Schließen der Verriegelung oder Verschraubung eine formschlüssige und lagestabile Verbindung erzielt wird und die Sende-und Empfangseinheit in der, während der Fertigung zuvor kalibrierten und auf die optische Achse des Bauteils justierten Lage, ohne weitere Justage fixiert werden kann.

11. Vorrichtung nach Anspruch 1 bis 10, **dadurch gekennzeichnet, dass** durch Analyse der Messsignale über der Zeit Defekte wie z.B. Defekte der Einspritzanlage oder der Luftzuführung oder der Dichtungen am Motor detektiert werden können, z.B. im Zusammenhang mit der signifikanten Abnahme der integralen Intensität am Sensor, infolge von Ruß oder anderen anormalen Absorptionen oder die durch das Gasgemisch im Abgasstrang verursachter verstärkter Streuung.

12. Vorrichtung nach einem der Ansprüche 1 bis 11 wobei die durch Auswertung des Messsignals ermittelte N02 Konzentration als Relationswert auch die Bestimmung der Konzentration oder das Vorhandensein weiterer Komponenten bzw. Eigenschaften des Abgases ermöglicht, indem z.B. mit Hilfe chemisch-physikalischer und thermodynamischer Modelle des Motors, des Verbrennungsvorgangs und ggf. der Abgasbehandlungsanlage bzw. der relationalen Zusammensetzung des Abgasstroms, diese Konzentrationen oder Eigenschaften bestimmt werden, so dass auf dieser aus dem Modell gewonnener Informationsbasis Optimierungen des Verbrennungsprozesses und des Betriebzustands des Motors z.B. in Echtzeit möglich sind, die z.B. zur Verbesserung des Wirkungsgrades oder zur Minimierung der Emissionen beitragen oder das frühzeitige Erkennen von Verschleiß oder Defekten ermöglichen, dabei können die Messwerte und die Ergebnisse der Modellrechnung verwendet werden das Modell dem Verschleiß bzw. der Alterung der durch das Modell dargestellten Komponenten nachzuführen.

13. Verfahren zur Bestimmung der Konzentration von $NO_2$ in Gasgemischen, insbesondere Abgasen von Dieselmotoren, bei welchem:

- mittels einer Lichtquelle Licht in einen Lichtweg eingekoppelt und über eine Sensoreinrichtung erfasst wird, wobei der Verlauf des Lichtweges so gewählt ist, dass dieser das zu untersuchende Gas durchsetzt, und wobei die Sensoreinrichtung so gestaltet ist, dass diese Signale liefert die das Spektrum des erfassten Lichtes beschreiben; und
- mittels einer Auswertungsschaltung jene durch die Sensoreinrichtung generierten Signale ausgewertet werden, wobei die Sensoreinrichtung innerhalb eines eingeschränkten spektralen Bereiches betrieben wird in welchem eine ausgeprägte absolute oder relative Variation des Absorptionskoeffizienten des $NO_2$ und zugleich eine geringe absolute oder relative Variation der weiteren Gase vorliegt.

14. Verfahren nach Anspruch 13, **dadurch gekennzeichnet, dass** nach Maßgabe der spektrometrisch ermittelten $NO_2$-Konzentration eine Abgasbehandlung abgewickelt wird.

15. Verfahren nach Anspruch 13 oder 14, **dadurch gekennzeichnet, dass** die vorgenannte Abgasbehandlung eine nach Maßgabe der ermittelten Konzentration dosierte $NH_3$ Einspeisung in ein Abgasbehandlungssystem umfasst.

Figur 1

(bl): NEW0029    Ref (gn): NEW0002

Figur 2

NEW0052   Ref: NEW0002

Figur 3

Senderoptik

optischer Pfad durch
entspr. Kompartiment

durchstimmbarer
Filter

Lichtquelle

Sensor

Fenster,
z.B.
katalytisch
beschichtet

Empfängeroptik

Steuer- und
Auswerteeinheit

Uni-Box mit
SCR-Kat etc.

# Figur 4

optischer Pfad

Filterrad mit Bandpass

Motor

Lichtquelle

Senderoptik

Fenster, z.B. katalytisch beschichtet

Empfängeroptik

Abgasstrom

Sensor

Steuer- und Auswerteeinheit

Messwerte und Daten zur Motorsteuerung

# Figur 5

Referenz-markierung

z.B. 500nm

Legende

z.B. 400nm

Filterrad mit Bandpass

Schritt-motor

Sensor

Empfängeroptik

# Figur 6

Referenzmarkierungen

Filterrad

Getriebe
-motor

Referenzmarkierung
Absolutposition

Sensor

Sensoroptik

z.B. 500nm

z.B. 400nm

Filteroptik

z.B. 500nm

Fenster mit
Bandpass

Legende

# Figur 7

Welle, z.B mit Treibrad

Motor

Filterrad

Sensor

Empfängeroptik

Markierungs-sensor

# Figur 8

Luftvorhang

optischer Pfad

FP-Filter mit
variabler Kavität

Lichtquelle

Sensor

Senderoptik

Empfängeroptik

Fenster, z.B.
katalytisch
beschichtet
mit
Luftvorhang

Abgasstrom

Steuer- und
Auswerteeinheit

Messwerte und Daten
zur Motorsteuerung

# Figur 9

Figur 10

Figur 11

Figur 12

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9953297 A
- US 4001103 A
- US 7156966 B
- DE 000019635977 A1
- DE 102007000028 A1
- EP 0816835 A
- DE 102007009824 A1

**In der Beschreibung aufgeführte Nicht-Patentliteratur**

- **Fumikazu Taketani ; Megumi Kawai ; Kenshi Takahashi ; Yutaka Matsumi.** Trace detection of atmospheric NO2 by laser-induced fluorescence using a GaN diode laser and a diode-pumped YAG laser. *Appl. Opt.,* 2007, vol. 46, 907-915
- **Wierzbowska et al.** *Optica Applicata,* 2005, vol. 35 (3), 655-662